(19) Europäisches Patentamt
European Patent Office
Office européen des brevets

(11) EP 4 570 862 A1

(12) EUROPEAN PATENT APPLICATION

(43) Date of publication:
**18.06.2025 Bulletin 2025/25**

(21) Application number: **24219035.3**

(22) Date of filing: **11.12.2024**

(51) International Patent Classification (IPC):
***C09C 1/00*** *(2006.01)* ***A61K 8/19*** *(2006.01)*
***C09D 5/36*** *(2006.01)* ***C09D 11/02*** *(2014.01)*

(52) Cooperative Patent Classification (CPC):
**C09C 1/0015; A61K 8/19; C09C 1/0021; C09C 1/0024; C09C 1/0039; C09C 1/0051; C09C 1/0066; C09D 5/36; C09D 11/037;**
C01P 2004/54; C01P 2004/61; C09C 2200/1004; C09C 2200/301; C09C 2200/302; C09C 2200/303;
(Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **12.12.2023 EP 23215984**

(71) Applicant: **Merck Patent GmbH**
**64293 Darmstadt (DE)**

(72) Inventors:
- **Iwabuchi, Atsuko**
  **Tokyo, 153-8605 (JP)**
- **Sasaki, Fumiko**
  **Tokyo, 153-8605 (JP)**
- **Kobayashi, Satoru**
  **Tokyo, 153-8605 (JP)**

(74) Representative: **Merck Patent Association**
**Merck Patent GmbH**
**64271 Darmstadt (DE)**

# (54) AL2O3 FLAKES

(57) The present invention relates to pearlescent pigments with a superior flip flop effect which are based on defined $Al_2O_3$ flakes and to the use thereof in paints, industrial coatings, automotive coatings, printing inks, cosmetic formulations and in particular as transparent substrate for effect pigments.

(52) Cooperative Patent Classification (CPC): (Cont.)
C09C 2200/305; C09C 2200/308; C09C 2210/20;
C09C 2220/10

## Description

The present invention relates to defined $Al_2O_3$ flakes and pearlescent pigments with a superior flip flop effect which are based on defined $Al_2O_3$ flakes and to the use thereof in paints, industrial coatings, automotive coatings, printing inks, cosmetic formulations and in particular as transparent substrate for effect pigments.

Imparting a pearlescent luster, metallic luster, color flop or multicolor effect can be achieved by using pearlescent pigments based on natural or synthetic transparent flakes like mica.

The key factors of a substrate suitable as a base substrate for an effect pigment are the particle size, shape, surface property, refractive index and the like. Since large and small particles have different proportions of reflection and transmission of light on the particle surface, uniformness in particle size is essential for a vivid and uniform color of the final effect pigment. Also, the particle size greatly affects the coloration of the pearlescent pigment because it is closely related with the wavelength of the light. That is, the smaller the particle size, the larger the surface area, thereby increasing the coloration and enhancing reflectivity, and offering a more vivid color. However, to apply a coating like a metal layer or metal oxide layer on the surface of $Al_2O_3$ flakes is very difficult because the coating is not uniform and thus results in a decrease in the aspect ratio, which then reduces the effect of light interference thus deteriorating glossiness of the resulting pearlescent colors.

The alumina flakes with the properties of the alumina flakes as such and of effect pigments based on alumina flakes can be increased by using alumina flakes with precisely defined dimensions and particle size and thickness distribution. Especially the optical properties of the alumina flakes and the effect pigments based on alumina flakes can be influenced by altering the particle size distribution.

In the literature alumina flakes with different size and thickness are well-known.

$\alpha$-$Al_2O_3$ in the form of hexagonal flakes having a particle diameter greater than 10 $\mu$m and an aspect ratio (particle diameter/thickness) of 5-10 is known from the Japanese Patent Publication No. 111239/1982.

The Japanese Patent Publication No. 72572/1991 discloses $\alpha$-$Al_2O_3$ in the form of flakes having an average particle diameter of 0.5-3 $\mu$m.

The Japanese Patent Publication No. JP 39362/1992 describes $Al_2O_3$ in the form of fine platy particles of a hexagonal crystal system with the plane perpendicular to the c axis grown into a plate.

$Al_2O_3$ flakes composed of aluminum oxide (as a major constituent) and of titanium dioxide (as a minor constituent) are disclosed in U.S. 5,702,519. The $Al_2O_3$ flakes have an average particle diameter of about 5-60 $\mu$m, a thickness less than 1 $\mu$m, and an aspect ratio of > 20.

Glitter pigments like pearlescent pigments based on alumina flakes are well known in the literature for example EP 2 799 387, EP 2 799 388, WO 2006/101306, WO 2008/026860 A1 and are commercially available under the trademarks Xirallic® from Merck KGaA and Adamas® from CQV.

But the $Al_2O_3$ flakes of the prior art have the disadvantages that they do not have sufficient thinness morphology and the required uniformity of their thickness distribution.

The objective of the present invention is to provide alumina flakes with high smoothness, high and deep glossiness, and good hiding power which show a superior flip flop effect when coated with at least one layer, for example a metal oxide layer.

It has now been found that alumina flakes as such and pearlescent pigments with a superior flip flop effect based on very thin alumina flakes can be obtained by using alumina flakes having a precisely defined particle size and thickness distribution.

Especially the optical properties of the alumina flakes and the effect pigments based on the alumina flakes are influenced by the particle thickness distribution of the alumina flakes.

Surprisingly, it has now been found that very thin and transparent alumina flakes having an average particle diameter of $D_{50}$ = 14-25 $\mu$m, a fraction of particle thickness of 40-130 nm greater than 40 % show improved optical properties and a high physical and chemical stability. The effect pigments based on these transparent alumina flakes show a high and deep luster combined with a superior flip flop.

Compared to the prior art, the coated $Al_2O_3$ flakes according to the present invention show in particular increased chroma, higher luster, lower haze and excellent finishing and at the same time a high chemical stability and high smoothness due to a less graininess.

The alumina flakes according to the invention are used, in particular, as substrates for effect pigments, especially for the use in industrial applications, plastics, automotive coatings including refinishing and cosmetics. Thus, they can also be employed in all formulations where coated or uncoated alumina flakes are usually employed, such as, for example, in inks, coatings, preferably automotive coatings, plastics, cosmetic formulations and as substrate for effect pigments.

The $Al_2O_3$ flakes of this invention have a particle size distribution characterized by a Gaussian distribution in which the volume size fractions are distributed as follows:

- $D_{50}$ is in the range of 14-25 $\mu$m, preferably 14-22 $\mu$m

- $D_{90}$ is in the range of 25-40 μm, preferably 25-35 μm.

In a preferred embodiment the $D_{10}$ value of the alumina flakes according to the present invention is < 9.5, preferably ≤ 9.0.
In a preferred embodiment the $D_{10}$ value is < 9.5, $D_{50}$ is 14-25 μm, preferably 14-22 μm and $D_{90}$ is 25-35 μm.
In this patent application $D_{10}$, $D_{50}$ and $D_{90}$ of the alumina flakes are evaluated by using Malvern MS 3000.
The particle size distribution $D_{50}$ is also known as the median diameter or the medium value of the particle size distribution, it is the value of the particle diameter at 50 % in the cumulative distribution and is one of the important parameters characterizing the particle size of the $Al_2O_3$ flakes.
Correspondingly, the $D_{90}$ value indicates the maximum longitudinal dimensions of the $Al_2O_3$ flakes, as determined again by means of laser granulometry in the form of sphere equivalents, which 90 % of the particles attain at maximum, or fall below, out of the entirety of all $Al_2O_3$ particles.
The $D_{10}$ value indicates the value of the longitudinal dimension of the $Al_2O_3$ flakes, as determined by means of laser granulometry in the form of the sphere equivalent, which 10 % of the flakes attain at most, or fall below, out of the entirety of all the $Al_2O_3$ flakes.
In a preferred embodiment, the $Al_2O_3$ flakes according to the present invention have a standard deviation of thickness distribution of less than 100, preferably less than 70 and in particular less than 50.
In this patent application the average thickness is determined on the basis of a cured paint film in which the $Al_2O_3$ flakes are oriented substantially plane-parallel to the substrate. For this purpose a transverse section of the cured paint film is examined under a scanning electron microscope (SEM), the thickness of 100 $Al_2O_3$ flakes being ascertained and statistically averaged.
The desired size and thickness distribution can be obtained by suitable classification of the flakes, such as by classifying through selected screens and the like.
The $Al_2O_3$ flakes according to the invention have a thickness of 40-130 greater than 40 %.
The $Al_2O_3$ flakes according to the invention preferably have an aspect ratio (diameter/thickness ratio) of 70-300, in particular of 120-250.
In a preferred embodiment the $Al_2O_3$ flakes of the present invention are $\alpha$-$Al_2O_3$ flakes.
The $Al_2O_3$ flakes can be prepared by methods known per se, as described in the literature.
In a preferred embodiment the $Al_2O_3$ flakes are prepared starting from an aqueous aluminum salt solution by precipitation with an aqueous alkali carbonate solution. An alkali metal salt like sodium or potassium sulphate and phosphoric acid or and phosphate as and optionally at least one dopant, for example a titanium, zirconium, silica, indium, tin, zinc, tungsten, molybdenum or indium compound are added to the starting solution. The precipitation step is followed by drying (evaporation, dehydration by heating), and molten salt treatment including the following steps:

(1) Preparation of an aqueous solution or a slurry of at least one water-soluble and/or insoluble aluminum salt,

(2) Adding an alkali solution to the aluminum salt solution to precipitate aluminum hydroxide particles, and adding a phosphorous compound and optionally at least one dopant to the aqueous solution before, during or after the precipitation,

(3) Evaporation of the water, followed by drying of the precipitated product of step (2) to form the dried form of alumina containing particle and alkali salt,

(4) Calcination, preferably at temperatures of 900 - 1400 °C, for 0.5-10 h, preferably 1-6 h, of the dried form obtained in step (3) to obtain $Al_2O_3$ flakes in the molten salt,

(5) Removing the water soluble part of the calcined material obtained in step (4),

(6) Adjust the particle size and thickness, for example by sieving, milling and/or sedimentation.

Examples for suitable aluminum salts are aluminum sulfate, aluminum chloride, aluminum nitrate, poly aluminum chloride, aluminum hydroxide, boehmite, basic aluminum sulfate and combinations thereof.
Examples for suitable alkali metal salts which function as mineralizer, include sodium sulfate, potassium sulfate, lithium sulfate, magnesium sulfate, sodium chloride and potassium chloride.
The phosphoric compound is preferably selected from phosphoric acid, phosphates, diphosphoric acid, sodium phosphate, ammonium phosphate dibasic and potassium phosphate. The amount of one or more phosphorous compound(s) is preferably 0.05-2 wt.% based on the alumina flakes.
The preferred example of the pH controlling agent for the precipitation is ammonia, sodium hydroxide, potassium

hydroxide, sodium carbonate, potassium carbonate and combinations thereof.

To control the particle size, thickness, optical properties and/or surface morphology it could be helpful to add one or more dopants in amounts of 0.01-5 wt.% based on the $Al_2O_3$ flake.

The dopant is preferably selected from the following group of compounds: $TiO_2$, $ZrO_2$, $SiO_2$, $In_2O_3$, $SnO_2$, $WO_3$, MoOs, ZnO and combinations thereof.

The $Al_2O_3$ flakes according to the present invention are highly suitable as substrate in the preparation of effect pigments. To this end, they are preferably coated with at least one high refractive index layer, like a layer of metal oxide, such as, for example, $TiO_2$, $ZrO_2$, $SnO_2$, ZnO, $Ce_2O_3$, $Fe_2O_3$, $Fe_3O_4$, FeTiOs, $Cr_2O_3$, CoO, $Co_3O_4$, $VO_2$, $V_2O_3$, NiO, furthermore of titanium suboxides ($TiO_2$ partially reduced with oxidation states from < 4 to 2, such as the lower oxides TisOs, $Ti_2O_3$, TiO), titanium oxynitrides, FeO(OH), thin semitransparent metal layers, for example comprising Al, Fe, Cr, Ag, Au, Pt or Pd, or combinations thereof. The $TiO_2$ layer may be in the rutile or anatase modification. In general, the highest quality and gloss and at the same time the most stable effect pigments are obtained when the $TiO_2$ is in the rutile modification. In order to obtain the rutile modification, an additive can be used which is able to direct the $TiO_2$ into the rutile modification. Useful rutile directors are disclosed in the U.S. 4,038,099 and U.S. 5,433,779 and EP 0 271 767. A preferred rutile director is $SnO_2$.

Preferred effect pigments based on $Al_2O_3$ flakes are coated with one or more layers of metal oxides, preferably with one metal-oxide layer only, in particular with $TiO_2$, $Fe_2O_3$, $Fe_3O_4$, $SnO_2$, $ZrO_2$ or $Cr_2O_3$. Especially preferred are $Al_2O_3$ flakes coated with $TiO_2$ or $Fe_2O_3$ and mixtures thereof.

The thickness of each high-refractive-index layer depends on the desired interference color. The thickness of each layer on the surface of the $Al_2O_3$ flakes is preferably 20-400 nm, preferably 30-300 nm, in particular 30-200 nm.

The number of layers on the surface of the $Al_2O_3$ flakes is preferably one or two, furthermore three, four, five, six or seven layers.

In particular, interference packages consisting of high- and low-refractive-index layers on the surface of the $Al_2O_3$ flakes result in effect pigments having increased gloss and a further increased interference color or superior flip flop effect.

Suitable colorless low-refractive-index materials for coating are preferably metal oxides or the corresponding oxide hydrates, such as, for example, $SiO_2$, $Al_2O_3$, AlO(OH), $B_2O_3$, compounds such as $MgF_2$ or a mixture of the said metal oxides.

In case of multilayers applied on the surface of the $Al_2O_3$ flakes the interference system is, in particular, a $TiO_2$-$SiO_2$-$TiO_2$ layer sequence.

Furthermore, the effect pigments according to the invention may also have a semitransparent metal layer as outer layer. Coatings of this type are known, for example, from DE 38 25 702 A1. The metal layers are preferably chromium or aluminum layers having layer thicknesses of 5-25 nm.

$Al_2O_3$ flakes can also be coated with one or more layers of a metal or metal alloy selected e.g. from chromium, nickel, silver, bismuth, copper, tin, or hastelloy. $Al_2O_3$ flakes coated with a metal sulfide are coated with sulfides e.g. of tungsten, molybdenum, cerium, lanthanum or rare earth elements.

Furthermore, the effect pigments based on $Al_2O_3$ flakes can be finally coated with an organic dye as a top coat, preferably with Prussian Blue or Carmine Red.

Particularly preferred effect pigments based on the $Al_2O_3$ flakes according to the invention have the following layer sequence(s):

$Al_2O_3$ flake + $TiO_2$
$Al_2O_3$ flake + $TiO_2/Fe_2O_3$
$Al_2O_3$ flake + $Fe_2O_3$
$Al_2O_3$ flake + $TiO_2$ + $Fe_2O_3$
$Al_2O_3$ flake + $TiO_2$ + $Fe_3O_4$
$Al_2O_3$ flake + $TiO_2$ + $SiO_2$ + $TiO_2$
$Al_2O_3$ flake + $Fe_2O_3$ + $SiO_2$ + $TiO_2$
$Al_2O_3$ flake + $TiO_2/Fe_2O_3$ + $SiO_2$ + $TiO_2$
$Al_2O_3$ flake + $TiO_2$ + $SiO_2$ + $TiO_2/Fe_2O_3$
$Al_2O_3$ flake + $TiO_2$ + $SiO_2$
$Al_2O_3$ flake + $TiO_2$ + $SiO_2/Al_2O_3$
$Al_2O_3$ flake + $TiO_2$ + $Al_2O_3$
$Al_2O_3$ flake + $SnO_2$
$Al_2O_3$ flake + $SnO_2$ + $TiO_2$
$Al_2O_3$ flake + $SnO_2$ + $Fe_2O_3$
$Al_2O_3$ flake + $SiO_2$
$Al_2O_3$ flake + $SiO_2$ + $TiO_2$
$Al_2O_3$ flake + $SiO_2$ + $TiO_2/Fe_2O_3$

$Al_2O_3$ flake + $SiO_2$ + $Fe_2O_3$
$Al_2O_3$ flake + $SiO_2$ + $TiO_2$ + $Fe_2O_3$
$Al_2O_3$ flake + $SiO_2$ + $TiO_2$ + $Fe_3O_4$
$Al_2O_3$ flake + $SiO_2$ + $TiO_2$ + $SiO_2$ + $TiO_2$
$Al_2O_3$ flake + $SiO_2$ + $Fe_2O_3$ + $SiO_2$ + $TiO_2$
$Al_2O_3$ flake + $SiO_2$ + $TiO_2/Fe_2O_3$ + $SiO_2$ + $TiO_2$
$Al_2O_3$ flake + $SiO_2$ + $TiO_2$ + $SiO_2$ + $TiO_2/Fe_2O_3$
$Al_2O_3$ flake + $SiO_2$ + $TiO_2$ + $SiO_2$
$Al_2O_3$ flake + $SiO_2$ + $TiO_2$ + $SiO_2/Al_2O_3$
$Al_2O_3$ flake + $SiO_2$ + $TiO_2$ + $Al_2O_3$
$Al_2O_3$ flake + $TiO_2$ + $SnO_2$ + $TiO_2$
$Al_2O_3$ flake + $TiO_2$ + Prussian Blue
$Al_2O_3$ flake + $TiO_2$ + Carmine Red
$Al_2O_3$ flake + Ag

The $TiO_2$ layer(s) in the preferred embodiments mentioned above can be in the rutile or anatase modification. The $Al_2O_3$ flakes mentioned above in the preferred embodiments can be doped or undoped.

In this application, the term "coating" or "layer" is taken to mean the complete enveloping of the $Al_2O_3$ flakes according to the invention.

The effect pigments based on doped or undoped $Al_2O_3$ flakes preferably consist of 40-90 wt.% of $Al_2O_3$ flakes and 10-60 wt.% of the coating based on the total pigment.

The $Al_2O_3$ flakes can be coated by wet chemical coating, by CVD or PVD processes.

The coating of the $Al_2O_3$ flakes with one or more layers, preferably one or more metal oxide layers, is preferably carried out by wet-chemical methods, it being possible to use the wet-chemical coating methods developed for the preparation of pearlescent pigments. Methods of this type are described, for example, in DE 14 67 468, DE 19 59 988, DE 20 09 566, DE 22 14 545, DE 22 15 191, DE 22 44 298, DE 23 13 331, DE 15 22 572, DE 31 37 808, DE 31 37 809, DE 31 51 343, DE 31 51 354, DE 31 51 355, DE 32 11 602, DE 32 35 017 or also in further patent documents and other publications known to the person skilled in the art.

In the case of wet coating, the $Al_2O_3$ flakes are suspended in water, and one or more hydrolysable metal salts are added at a pH which is suitable for hydrolysis, which is selected in such a way that the metal oxides or metal-oxide hydrates are precipitated directly onto the flakes without secondary precipitations occurring. The pH is usually kept constant by simultaneous metered addition of a base and/or acid. The pigments are subsequently separated off, washed and dried at 50-150 °C for 6-18 h and calcined for 0.5-3 h, where the calcination temperature can be optimized with respect to the respective coating present. In general, the calcination temperatures are 500 - 1000 °C, preferably 600 - 900 °C. If desired, the pigments can be separated off after application of individual coatings, dried and optionally calcined and then re-suspended again for the application of the further layers.

The application of a $SiO_2$ layer to the $Al_2O_3$ flake and/or to the already coated $Al_2O_3$ flake is generally carried out by addition of a potassium or sodium water-glass solution at a suitable pH.

Furthermore, the coating can also be carried out in a fluidized-bed reactor by gas-phase coating, it being possible to use, for example, the methods proposed in EP 0 045 851 and EP 0 106 235 for the preparation of pearlescent pigments correspondingly.

The hue and chroma of the effect pigment based on $Al_2O_3$ flakes according to the invention can be varied in very broad limits through the different choice of the coating amounts or the layer thicknesses resulting therefrom. Fine tuning for a certain hue and or chroma can be achieved beyond the pure choice of amount by approaching the desired color under visual or measurement technology control.

In order to increase the light, water and weather stability, it is frequently advisable, depending on the area of application, to subject the final pigment to post-coating or post-treatment. Suitable post-coatings or post-treatments are, for example, the processes described in 22 15 191 C2, DE-A 31 51 354, DE-A 32 35 017 or DE-A 33 34 598. This post-coating further increases the chemical and photochemical stability or simplifies the handling of the pigment, in particular the incorporation into various media. In order to improve the weatherability, dispersibility and/or compatibility with the user media, it is possible, for example, for functional coatings of $Al_2O_3$ or $SiO_2$ ,$ZrO_2$ or mixtures thereof. Furthermore, the protective layer can contain an organic or mixtures thereof to be applied to the pigment surface. Furthermore, organic post-coatings are possible, for example with silanes, as described, for example, in EP 0090259, EP 0 634 459, WO 99/57204, WO 96/32446, WO 99/57204, U.S. 5,759,255, U.S. 5,571,851, WO 01/92425 or in J.J. Ponjeé, Philips Technical Review, Vol. 44, No. 3, 81 ff. and P.H. Harding J.C. Berg, J. Adhesion Sci. Technol. Vol. 11 No. 4, pp. 471-493.

Preferred metal oxides present in the post-treatment or protective layer are $Al_2O_3$, $SiO_2$, $ZrO_2$ and/or $Ce_2O_3$. Furthermore, the protective layer can contain an organic component in particular selected from organic coupling agents, organofunctional silanes, amino compounds, organic phosphorus compounds.

Suitable coupling reagents are for example, organosilanes, organoaluminates, organotitanates and/or zirconates. The coupling agents are preferably organosilanes.

Examples of organosilanes are propyltrimethoxysilane, propyltriethoxysilane, isobutyltrimethoxysilane, n-octyltrimethoxysilane, i-octyltrimethoxysilane, n-octyltriethoxysilane, n-decyltrimethoxysilane, dodecyltrimethoxysilane, hexadecyltrimethoxysilane, vinyltrimethoxysilane, preferably n-octyltrimethoxysilane and n-octyltriethoxysilane. Suitable oligomeric, alcohol-free organosilane hydrolysates are, inter alia, the products marketed under the trade name Dynasylan® Hydrosil by Evonik Industries, such as, for example, Dynasylan® Hydrosil 2926, Dynasylan® Hydrosil 2909, Dynasylan® Hydrosil 2907, Dynasylan® Hydrosil 2781, Dynasylan® Hydrosil 2776, Dynasylan® Hydrosil 2627. In addition, oligomeric vinylsilane and also aminosilane hydrolysate is suitable as organic coating. Functionalized organosilanes are, for example, 3-aminopropyltrimethoxysilane (AMMO), 3-methacryloxytrimethoxysilane (DAMO), 3-glycidyloxypropyltrimethoxysilane (GLYMO), beta-(3,4-epoxycyclohexyl)ethyltrimethoxysilane, gamma-isocyanatopropyltrimethoxysilane, 1,3-bis(3-glycidoxypropyl)-1,1,3,3,-tetramethyldisiloxane, ureidopropyltriethoxysilane, preferably 3-aminopropyltrimethoxysilane, 3-methacryloxytrimethoxysilane, 3-glycidyloxypropyl-trimethoxysilane, beta-(3,4-epoxycyclohexyl) ethyltrimethoxysilane, gamma-isocyanatopropyltrimethoxysilane. Examples of polymeric silane systems are described in WO 98/13426 and are marketed, for example, by Evonik Industries under the trade name Dynasylan® Hydrosil. The amount of organic coating can be between 0.2 and 5% by weight, based on the effect pigment, preferably 0.5 to 2% by weight.

Suitable coupling agents are among others zirconium aluminates of the following structure:

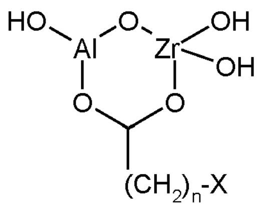

in which

X denotes $NH_2$, COOH, -COO-, hydroxyphenyl, methacrylate, carboxyphenyl, alkyl, mercapto, phenyl, H, vinyl, styryl, melamin, epoxy, aryl or alkyl

n denotes 0, 1, 2, 3, 4, 5, 6, 7, 8, 9, 10, 11 or 12.

Other suitable coupling reagents are metal acid esters of the following structure

$$M^n(OR)_y$$

wherein

M denotes Zr, Ti or Al,
N denotes the valence of the metal,
y 1, 2 or 3 depending on the valence of the metal
R denotes

(i) alkyl of 1-12 carbon atoms or aryl,
(ii)alkyl or aryl substituted by $-N(alkyl)_3$, $-NH(alkyl)_2$, $-NH_2(alkyl)$, $-NH_3$, $N(aryl)_3$, $-NH(aryl)_2$ or $-NH_2(aryl)$, where aryl can be substituted by halogen, nitro, amino or hydrogen,
(iii) -C-aryl or C-alkyl.

Particularly suitable metal acid esters for example acrylate functional and methacrylamide-functional titanates and methacrylamide-functional zirconates are commercially available.

In a preferred embodiment the proportion of the total amount of effect pigment made up by the protective layer on surface of the coated $Al_2O_3$ flakes is 2 to 20 wt.%, preferably 2 to 10 wt.% and in particular 2 to 5 wt.%. The composition of a very preferred protective layer as such contains 0.2 - 2 wt.% of a rare earth metal oxide, preferably $Ce_2O_3$, 0.2 - 2 wt. % of $SiO_2$ and 0.2 - 4 wt.% of $Al_2O_3$ and/or $ZrO_2$ and 1 - 10 wt.% of an organic component. In a preferred embodiment the organic component is a coupling agent.

In a particular preferred embodiment, the protective layer consists of 0.4 -1.5 wt.% of $Ce_2O_3$, 0.4 -1 wt. % of $SiO_2$ and 0.5 - 2.5 wt.% of $Al_2O_3$ and/or $ZrO_2$ and 2 - 5 wt.% of the coupling agent.

The protective coating on the effect pigments according to the invention is prepared by methods known to the skilled person. In a preferred embodiment the effect pigments are pre-treated by wet chemical coating.

In a preferred embodiment the effect pigments according to the invention have a specific surface area of ≤ $10m^2/g$, preferably ≤ 7 $m^2/g$, measured by the BET method (DIN ISO 9277: 2003-05).

The $Al_2O_3$ flakes and the effect pigments based on $Al_2O_3$ flakes according to the invention are compatible with a multiplicity of color systems, preferably from the area of paints, automotive coatings, industrial coatings, and printing inks and cosmetic formulations. For the preparation of printing inks for, for example, gravure printing, flexographic printing, offset printing and offset over varnishing, a multiplicity of binders, in particular water-soluble grades, as sold, for example, by BASF, Marabu, Pröll, Sericol, Hartmann, Gebr. Schmidt, Sicpa, Aarberg, Siegberg, GSB-Wahl, Follmann, Ruco or Coates Screen INKS GmbH, is suitable. The printing inks can be water-based or solvent-based. The pigments are furthermore also suitable for the laser marking of paper and plastics and for applications in the agricultural sector, for example for greenhouse sheeting, and, for example, for the coloring of tent awnings.

It goes without saying that, for the various applications, the $Al_2O_3$ flakes and the effect pigments according to the present invention can also advantageously be used in blends with organic dyes, organic pigments or other pigments, such as, for example, transparent and opaque white, colored and black pigments, and with flake-form iron oxides, holographic pigments, LCPs (liquid crystal polymers) and conventional transparent, colored and black luster pigments based on metal oxide-coated mica and $SiO_2$ flakes, etc. The $Al_2O_3$ flakes and effect pigments based on $Al_2O_3$ flakes according to the invention can be mixed in any ratio with commercially available pigments and fillers.

Fillers which may be mentioned are, for example, natural and synthetic mica, nylon powder, pure or filled melamine resins, talc, $SiO_2$, glasses, kaolin, oxides or hydroxides of aluminum, magnesium, calcium or zinc, BiOCl, barium sulfate, calcium sulfate, calcium carbonate, magnesium carbonate, carbon, and physical or chemical combinations of these substances. There are no restrictions regarding the particle shape of the filler. It can be, for example, flake-form, spherical or needle-shaped in accordance with requirements.

The $Al_2O_3$ flakes and effect pigments based on $Al_2O_3$ flakes according to the invention are simple and easy to handle. The $Al_2O_3$ flakes and the effect pigments based on $Al_2O_3$ flakes can be incorporated into the system in which it is used by simple stirring. Laborious milling and dispersing of the $Al_2O_3$ flakes and the effect pigments is not necessary.

The effect pigments based on $Al_2O_3$ flakes according to the invention can be used for pigmenting coating materials, printing inks, plastics, agricultural films, button pastes, for the coating of seed, for the coloring of food, coatings of medicaments or cosmetic formulations.

The concentration of the $Al_2O_3$ flakes and the effect pigments in the system in which it is to be used for pigmenting is generally between 0.01 and 50 % by weight, preferably between 0.1 and 5 % by weight, based on the overall solids content of the system. This concentration is generally dependent on the specific application.

Plastics containing the $Al_2O_3$ flakes and effect pigments according to the invention in amounts of 0.1 to 50 % by weight, in particular from 0.5 to 7 % by weight, are frequently notable for a particular gloss effect.

In the coating sector, especially in automotive coatings and automotive finishing, the $Al_2O_3$ flakes and effect pigments according to the invention are employed in amounts of 0.5-10 % by weight.

In the coating material, the effect pigments according to the invention have the advantage that the desired color and gloss is obtained by a single-layer coating (one-coat systems or as a base coat in a two-coat system).

The invention likewise provides pigment preparations containing coated or uncoated $Al_2O_3$ flakes according to the present invention and further effect pigments, binders and, if desired, additives, the said preparations being in the form of substantially solvent-free, free-flowing granules. Such granules contain up to 95 % by weight of the effect pigment according to the invention. A pigment preparation in which the $Al_2O_3$ flakes and the effect pigments based on $Al_2O_3$ flakes according to the invention is pasted up with a binder and with water and/or an organic solvent, with or without additives, and the paste is subsequently dried and brought into a compact particulate form, e.g. granules, pellets, briquettes, a masterbatch or tablets, is particularly suitable as a precursor for printing inks.

The invention thus also relates to the use of coated $Al_2O_3$ flakes or uncoated $Al_2O_3$ flakes in formulations from the areas of paints, glaze compositions, coatings, automobile coatings, automotive finishing, industrial coatings, paints, powder coatings, printing inks, security printing inks, plastics, ceramic materials, cosmetics. The coated and uncoated $Al_2O_3$ flakes can furthermore be employed in glasses, in paper, in paper coating, in toners for electrophotographic printing processes, in seed, in greenhouse sheeting and tarpaulins, in thermally conductive, self-supporting, electrically insulating, flexible sheets for the insulation of machines or devices, as absorber in the laser marking of paper and plastics, as absorber in the laser welding of plastics, in pigment pastes with water, organic and/or aqueous solvents, in pigment preparations and dry preparations, such as, for example, granules, for example in clear coats in the industrial and automobile sectors, in sunscreens, as filler, in particular in automobile coatings and automotive refinishing, for LIDAR and RADAR applications.

In this patent application the term "coated alumina" flakes means alumina flakes according to Claim 1 which are coated on the surface with one or more layers as mentioned above. The alumina flakes according to Claim 1 are suitable as substrates for effect pigments.

All percentage data in this application are per cent by weight, unless indicated otherwise.

The following examples are intended to explain the invention in greater detail, but without restricting it. Above and below, all percentages are per cent by weight.

**Examples**

Example 1: $TiO_2$ coated alumina flakes

Example 1a: Alumina flakes preparing process

In 1200 ml of deionized water are dissolved 223.8 g of aluminum sulfate 18-hydrate ($Al_2(SO_4)_3$- 18 $H_2O$) and 286.3 g of anhydrous sodium sulfate ($Na_2SO_4$), by heating up to about 75 °C. To the resulting solution is added 0.6 g of 34.4 % solution of titanyl sulfate ($TiOSO_4$). The resulting solution is designated as the aqueous solution (a).

In 550 ml of deionized water are dissolved 0.9 g of sodium tertiary phosphate 12-hydrate ($Na_3PO_4 \cdot 12\ H_2O$) and 107.9 g of sodium carbonate ($Na_2CO_3$). The resulting solution is designated as the aqueous solution (b).

The aqueous solutions (a) and (b) are added simultaneously to 550 ml of deionized water with stirring at a constant rate over about 15 minutes, in such a manner that the solutes in the solution (a) are approximately equivalent to the solutes in the solution (b). Stirring is continued for additional 15 minutes.

The resulting solution is evaporated to dryness. The resulting solids are heated at 1000 °C for 5 hours. Water is added to the heat-treated product to dissolve free sulfate. Insoluble solids are filtered off, washed with water, and finally dried. Thus, there is obtained the desired flaky aluminum oxide having an average particle diameter of 14.3 μm and 89 % fraction of particle thickness of 40 - 130 nm.

Example 1b: $TiO_2$ coating process on alumina flakes

20 g alumina flakes of Example 1a are suspended in 400 ml of deionized water. To the resulting suspension (kept at about 65 °C) is added a solution containing 125 g of $TiCl_4$ per liter. Simultaneously a 10 % solution of NaOH is added to keep the pH at 2.1. The addition of the $TiCl_4$ solution is stopped when the resulting product takes on a silvery color. The suspending solids are filtered off, washed with water, and dried. Finally, the dried solids are calcined at 850 °C for 30 minutes to give a whitish and a little glossy pearlescent pigment. The obtained effect pigments show a superior flip flop effect which is represented by the Flop Index: 6.0.

Example 1c: Surface treatment

100 g of alumina flakes coated with $TiO_2$ according to Example 1b are suspended in 1000 ml of deionized water. To the resulting suspension, kept at about 70 °C. and adjusted to pH 7 with 10% HCl, is added 100 ml of a water glass solution containing 3.05 g $Na_2SiO_3$ during 90 min. Simultaneously a 10 % solution of HCl is added to keep the pH at 7. Subsequently 100 ml of a solution containing 6.5 g aluminum sulfate ($Al_2(SO4)_3$*18 $H_2O$) is added during 90 min to the suspension while keeping the pH to 7with 10 % NaOH. After raising the pH to 7.5 with 10 % NaOH 1.5 g of 3-aminopropyl-trimethoxysilane (CAS No. 13822-56-5) and 1.5 g 3-glycidyloxypmpyl-trimethoxysilane (CAS No.2530-83-8) are added subsequently during 15 min each to the suspension while keeping the pH at 7.0 with 10 % HCl or 10 % NaOH. The suspending solids are filtered off, washed with water, dried at 140° C. and sieved (325 mesh).

The surface-treated effect pigment shows a good humidity resistance due to remaining photoactivity.

Example 1d: Sprayed panel paint process

A base coat paint for automobiles is prepared according to the following formulation.

| <Base coat system> Acrylic-melamine resin system | |
|---|---|
| "Acrydic® 47-712" * | 70 pbw |
| "Superbekkamine® G821-60" ** | 30 pbw |
| Toluene | 30 pbw |
| Ethyl acetate | 50 pbw |
| * Acrylic resin from Dainippon Ink & Chemicals, Inc.<br>** Melamine resin from Dainippon Ink & Chemicals, Inc | |

| <Base coat system> Acrylic-melamine resin system | |
|---|---|
| n-Butanol | 110 pbw |
| Solvesso® #150 | 40 pbw |

The above acrylic-melamine resin system (100 pbw) is incorporated with 20 pbw of the flaky aluminum oxide (Example 1a) or pearlescent pigment obtained in Example 1b. The resulting compound is diluted with a thinner so that the resulting paint has an adequate consistency for spraying. (12-15 seconds, Ford cup #4) This paint is applied to a substrate by spraying to form a base coat layer.

The base coated layer is coated further with a colorless top clear coat paint, which is prepared according to the following formulation.

| <top claer coat system> | |
|---|---|
| "Acrydic® 44-179" | 14 pbw |
| "Superbekkamine® L117-60" | 6 pbw |
| Toluene | 4 pbw |
| MIBK | 4 pbw |
| Butyl cellosolve | 3 pbw |

The top coating is exposed to air at 40 °C for 30 minutes and then cured at 135 °C for 30 minutes.

Example 2: $TiO_2$ coated alumina flakes

The preparation of the alumina flakes is conducted according to Example 1a but the amount of anhydrous sodium sulfate is 429.4 g. The obtained alumina flakes have an average particle diameter greater than 15.1 μm and 93 % fraction of particle thickness is in the range of 40-130 nm

The $TiO_2$ coating process on alumina flakes is conducted according to Example 1b. The obtained effect pigments show a superior flip flop effect which is represented by the Flop Index: 6.6.

The sprayed panel paint process is conducted according to Example 1d.

Example 3: $TiO_2$ coated alumina flakes

1261.7 g of 27 % aluminum sulfate solution as $Al_2(SO_4)_3$, 512.0 g of sodium sulfate ($Na_2SO_4$), 415.6 g of potassium sulfate ($K_2SO_4$) and 6.0 g of a 35 % zinc sulfate ($ZnSO_4 \cdot 7H_2O$) solution is added to a 5 l reactor containing 1200 ml of pure water. A homogeneous mixture solution is obtained by mixing at 65 °C. An alkali solution is prepared by dissolving 318.7g of sodium carbonate ($Na_2CO_3$) and 2.7 g of sodium phosphate ($(NaPO_3)_6$) in 896.5 ml of distilled water at 65 °C. The aluminum sulfate mixture solution is titrated with the alkali solution at a rate of 20 ml/min, while stirring, to pH 6.8. A gel mixture of pseudo-boehmite and flux is obtained. Then, the gel mixture is aged at 90 °C for 20 hrs, distilled under vacuum at 60 °C and dried at 110 °C for 20 hrs.

The resulting solution is evaporated to dryness. The resulting solids are heated at approximately 1150 °C for 5 hours. Water is added to the heat-treated product to dissolve free sulfate. Insoluble solids are filtered off, washed with water, and finally dried. Thus, there is obtained the desired flaky aluminum oxide.

The obtained alumina flakes have an average particle diameter greater than 16.5 μm and a 45 % fraction of particle thickness of 40-130 nm.

The $TiO_2$ coating process on alumina flakes is conducted according to Example 1b. The obtained effect pigments show a superior flip flop effect which is represented by the Flop Index: 4.3.

The sprayed panel paint process is conducted according to Example 1d.

Example 4: $TiO_2$ coated alumina flakes

The alumina flakes are prepared according to Example 1a but the amount of a 35 % zinc sulfate ($ZnSO_4 \cdot 7H_2O$) solution is 23.9 g.

The obtained alumina flakes have an average particle diameter greater than 18.3 μm and a 42 % fraction of particle thickness of 40-130 nm.

The $TiO_2$ coating process on alumina flakes is conducted according to Example 1b. The obtained effect pigments

show a superior flip flop effect which is represented by the Flop Index: 4.5.

The sprayed panel paint process is conducted according to Example 1d.

Example 5: $TiO_2$ coated alumina flakes

Homogeneous mixture solution is prepared by mixing 423.2 g of aluminum sulfate ($Al_2(SO_4)_3 \cdot 18\ H_2O$), 326.8 g of sodium sulfate ($Na_2SO_4$), 265.3 g of potassium sulfate ($K_2SO_4$), 3.0 g of 34 % zinc sulfate ($ZnSO_4 \cdot 7H_2O$) aqueous solution and 1.0 g of 17 % tin sulfate ($SnSO_4 \cdot 7H_2O$) aqueous solution in a reactor (5 l) containing 1,200 ml of purified water at 65 °C. Alkaline solution is prepared by dissolving 204.6g of sodium carbonate ($Na_2CO_3$) and 1.7 g of sodium phosphate ($(NaPO_3)_6$) in 568.4 ml of distilled water at 65 °C. A gel mixed with pseudo-boehmite and flux is prepared by the titration of the aluminum sulfate mixture solution (65 °C) while stirring with the alkaline solution at the rate of 25 ml/min to adjust the final pH to 6.8. The mixed gel is aged at 90 °C for 20 hours, vacuum-distilled at 60 °C, and dried at 110 °C for 20 hours.

The resulting solution is evaporated to dryness. The resulting solids are heated at approximately 1000 °C for 5 hours. Water is added to the heat-treated product to dissolve free sulfate. Insoluble solids are filtered off, washed with water, and finally dried. Thus, there is obtained the desired flaky aluminum oxide.

The obtained alumina flakes have an average particle diameter of greater than 16.0 μm and a 52 % fraction of particle thickness of 40-130 nm.

The $TiO_2$ coating process on alumina flakes is conducted according to Example 1b. The obtained effect pigments show a superior flip flop effect which is represented by the Flop Index: 5.2.

The sprayed panel paint process is conducted according to Example 1d.

Example 6: $TiO_2$ coated alumina flakes

Example 6 is prepared according to Example 1a but the amount of a 35 % zinc sulfate ($ZnSO_4 \cdot 7H_2O$) solution is 23.9 g.

The obtained alumina flakes have an average particle diameter greater than 15.5 μm and a 58 % fraction of particle thickness of 40-130 nm.

The $TiO_2$ coating process on alumina flakes is conducted according to Example 1b. The obtained effect pigments show a superior flip flop effect which is represented by the Flop Index: 5.4.

The sprayed panel paint process is conducted according to Example 1d.

Comparative Example 1: $TiO_2$ coated alumina flakes

In 1200 ml of deionized water are dissolved 596.8 g of aluminum sulfate 18-hydrate and 253.4 g of anhydrous sodium sulfate, by heating up to about 75 °C. To the resulting solution is added 1.6 g of 34.4 % solution of titanyl sulfate. The resulting solution is designated as the aqueous solution (a).

In 550 ml of deionized water are dissolved 4.9 g of sodium tertiary phosphate 12-hydrate and 287.7 g of sodium carbonate. The resulting solution is designated as the aqueous solution (b).

The aqueous solutions (a) and (b) are added simultaneously to 500 ml of deionized water with stirring at a constant rate over about 15 minutes, in such a manner that the solutes in the solution (a) are approximately equivalent to the solutes in the solution (b). Stirring is continued for additional 15 minutes.

The resulting solution is evaporated to dryness. The resulting solids are heated at approximately 1000 °C for 5 hours. Water is added to the heat-treated product to dissolve free sulfate. Insoluble solids are filtered off, washed with water, and finally dried and alumina oxide flakes are obtained.

The obtained alumina flakes have an average particle diameter greater than 12.2 μm and a 78 % fraction of particle thickness of 40-130 nm.

The $TiO_2$ coating process on alumina flakes is conducted according to Example 1b. The obtained effect pigments do not show any significant flip flop effect. Flop Index is only 3.7.

The sprayed panel paint process is conducted according to Example 1d.

Comparative Example 2: $TiO_2$ coated alumina flakes

In 1200 ml of deionized water are dissolved 596.8 g of aluminum sulfate 18-hydrate, 111.4 g of anhydrous sodium sulfate, and 177.5 g of potassium sulfate, by heating up to about 75 °C. To the resulting solution is added 5.3 g of 34.4 % solution of titanyl sulfate. The resulting solution is designated as the aqueous solution (a).

In 550 ml of deionized water are dissolved 2.4 g of sodium tertiary phosphate 12-hydrate and 287.7 g of sodium carbonate. The resulting solution is designated as the aqueous solution (b).

The aqueous solutions (a) and (b) are added simultaneously to 500 ml of deionized water with stirring at a

constant rate over about 15 minutes, in such a manner that the solutes in the solution (a) are approximately equivalent to the solutes in the solution (b). Stirring is continued for additional 15 minutes.

The resulting solution is evaporated to dryness. The resulting solids are heated at approximately 1200 °C for 5 hours. Water is added to the heat-treated product to dissolve free sulfate. Insoluble solids are filtered off, washed with water, and finally dried and alumina flakes are obtained.

The obtained alumina flakes have an average particle diameter greater than 19.7 μm and 2 % fraction of particle thickness between 40-130nm.

The $TiO_2$ coating process on alumina flakes is conducted according to Example 1b. The obtained effect pigments do not show a significant flip flop effect. Flop index is only 3.1.

The sprayed panel paint process is conducted according to Example 1d.

Comparative Example 3: $TiO_2$ coated alumina flakes

In 1200 ml of deionized water are dissolved 223.8 g of aluminum sulfate 18-hydrate and 334.3g of anhydrous sodium sulfate, by heating up to about 75°C. The resulting solution is designated as the aqueous solution (a).

In 550 ml of deionized water are dissolved 107.9 g of sodium carbonate. The resulting solution is designated as the aqueous solution (b).

The aqueous solutions (a) and (b) are added simultaneously to 500 ml of deionized water with stirring at a constant rate over about 15 minutes, in such a manner that the solutes in the solution (a) are approximately equivalent to the solutes in the solution (b). Stirring is continued for additional 15 minutes.

The resulting solution is evaporated to dryness. The resulting solids are heated at approximately 1000°C for 5 hours. Water is added to the heat-treated product to dissolve free sulfate. Insoluble solids are filtered off, washed with water, and finally dried and alumina flakes are obtained.

The alumina flakes have an average particle diameter greater than 13.0 μm and 22 % fraction of particle thickness of 40-130 nm.

The $TiO_2$ coating process on alumina flakes is conducted according to Example 1b. The obtained effect pigments show little or no flip flop effect. Flop index is only 1.9.

The sprayed panel paint process is conducted according to Example 1d.

Table 1: Color effects of the effect pigments according to Examples

| Examples | Alumina flakes | | Sprayed panels | | |
|---|---|---|---|---|---|
| | Particle Size D50 | Thickness 40-130 nm | L* 15° | Sparkle Grade (SG) value | Flop Index (FI) |
| | μm | % | | | |
| Ex.1 | 14.3 | 89% | 127.1 | 3.9 | 6.0 |
| Ex. 2 | 15.1 | 93% | 131.5 | 4.2 | 6.6 |
| Ex. 3 | 16.5 | 45% | 125.1 | 5.1 | 4.3 |
| Ex.4 | 18.3 | 42% | 124.3 | 6.4 | 4.5 |
| Ex. 5 | 16.0 | 52% | 125.4 | 4.9 | 5.2 |
| Ex. 6 | 15.5 | 58% | 125.9 | 4.8 | 5.4 |
| Comp.Ex.1 | 12.2 | 78% | 120.1 | 2.8 | 3.7 |
| Comp.Ex.2 | 19.7 | 2% | 122.5 | 7.4 | 3.1 |
| Comp.Ex.3 | 10.3 | 22% | 90.7 | 1.1 | 1.9 |

L*(15°): This value means the lightness which is measured with BYK-mac i(*). At this angle(15°),this value mainly indicates the luster. The higher the value, the higher the luster.

Sparkle Grade (SG): This value mainly indicates their glitter effect. The higher the value, the higher the glitter effect. This value is measured with BYK-mac i.

Flop Index (FI): This value mainly indicates their flip-flop effect. The higher the value, the higher the flip flop effect. This value is measured with BYK-mac i.

(*) BYK-mac i : Spectrophotometer of color and effect measurement of metallics and pearls. (BYK Additives & Instruments)

Table 1 shows that the effect pigments based on alumina flakes according to the present invention show a much

higher luster combined with a much higher color flip flop. Compared to the alumina based pigments of the prior art the effect pigments based on the inventive alumina flakes show a relatively low glitter effect compared to their particle size:
The effect pigments according to the present invention show a Flop Index of > 4 which means the relative change in brightness seen between around the reflection angle and around the recession angle, i.e., the greater the amount of change, the greater the sense of flop (color change with viewing angle).

Flop Index measures the change in brightness of a metallic color when it is tilted at all angles in the field of view. It scales the change in brightness when the metallic color is tilted at all angles in the field

Measurements:

Evaluation for particle size $D_{50}$

$D_{50}$ of the alumina flakes is evaluated by using Malvern MS3000.

Determination of the thickness and particle size and the thickness distribution

0.01 g/l of the alumina flake slurry is prepared, and 0.1 ml of this slurry is dropped onto a flat substrate like a silicon wafer.

The substrate is dried and cut to adequate size.

The substrate is set with almost vertically tilted angle on the base of SEM (Scanning electronic microscope) and the thickness of the alumina flake is determined.

The thickness of more than 100 alumina flakes is measured for the calculation of the thickness distribution.

L*(15°)

L* value at 15° in Figure 1 is measured with spectrophotometer BYK-mac i.

SG Value

The value is calculated by the following formula using the index value measured with BYK-mac i.

$$S_G = 0.35 * \sqrt{(S_i * S_a)} - 0.8$$

Si value = Sparkle intensity
Sa value = Sparkle area

(The impression of brilliance changes depending on the lighting angle. Therefore, BYK-mac i uses a very bright LED to illuminate the sample surface at three angles of 15° / 45° / 75° and takes an image with a vertically arranged CCD chip. Then, to calculate this image with the light brightness parameter, it is analyzed by an image analysis algorithm using a brightness level histogram, and it is analyzed in two dimensions to improve the identification accuracy. The calculated value is expressed numerically as the Sparkle area (Sa value) and the Sparkle intensity (Si value) at each angle.)

Flop index value

The FI value is calculated by the following formula using the index value measured with BYK-mac i.

$$FI = 2.69 \times \frac{(L^*15° - L^*110°)^{1.11}}{L^*45°^{\ 0.86}}$$

**Claims**

1. $Al_2O_3$ flakes having an average particle diameter of 14-25 μm and a fraction of particle thickness of 40-130 nm greater than 40 %.

2. $Al_2O_3$ flakes according to Claim 1 **characterised in that** the $Al_2O_3$ flakes are α-alumina flakes.

**3.** $Al_2O_3$ flakes according to Claim 1 or 2 **characterised in that** the $Al_2O_3$ flakes are doped with one or more oxides.

**4.** $Al_2O_3$ flakes according to one or more of Claims 1 to 3, **characterised in that** the amount of doping is 0.01 - 5 % by weight based on the $Al_2O_3$ flake.

**5.** $Al_2O_3$ flakes according to one or more of Claims 1 to 4, **characterised in that** the $Al_2O_3$ flakes are doped with one or more oxides selected from the group of $TiO_2$, $ZrO_2$, $SiO_2$, $SnO_2$, $In_2O_3$, ZnO, $WO_3$, MoOs and combinations thereof.

**6.** $Al_2O_3$ flakes according to one or more of Claims 1 to 5, **characterised in that** the $Al_2O_3$ flakes are coated with at least one layer of

- a metal oxide
- a mixture of at least two metal oxides
- a metal
- a metal sulfide
- a titanium suboxide
- a titanium oxynitride
- an organic or inorganic dye
- FeO(OH)
- SiO2
- a metal alloy
- a rare earth compound

or combinations thereof.

**7.** $Al_2O_3$ flakes according to one or more of Claims 1 to 6 **characterised in that** the $Al_2O_3$ flakes are coated with at least one layer of a metal oxide or a mixture of at least two metal oxides.

**8.** $Al_2O_3$ flakes according to one or more of Claims 1 to 7 **characterised in that** metal oxide is selected from the following group of oxides $TiO_2$, $Fe_2O_3$, $Fe_3O_4$, $SiO_2$, $SnO_2$, $Al_2O_3$, $ZrO_2$, $CeO_2$, $In_2O_3$, and ZnO or combinations thereof.

**9.** $Al_2O_3$ flakes according to one or more of Claims 1 to 8 **characterised in that** the $Al_2O_3$ flakes are coated with the following layer sequence:

$Al_2O_3$ flake + $TiO_2$
$Al_2O_3$ flake + $TiO_2/Fe_2O_3$
$Al_2O_3$ flake + $Fe_2O_3$
$Al_2O_3$ flake + $TiO_2$ + $Fe_2O_3$
$Al_2O_3$ flake + $TiO_2$ + $Fe_3O_4$
$Al_2O_3$ flake + $TiO_2$ + $SiO_2$ + $TiO_2$
$Al_2O_3$ flake + $Fe_2O_3$ + $SiO_2$ + $TiO_2$
$Al_2O_3$ flake + $TiO_2/Fe_2O_3$ + $SiO_2$ + $TiO_2$
$Al_2O_3$ flake + $TiO_2$ + $SiO_2$ + $TiO_2/Fe_2O_3$
$Al_2O_3$ flake + $TiO_2$ + $SiO_2$
$Al_2O_3$ flake + $TiO_2$ + $SiO_2/Al_2O_3$
$Al_2O_3$ flake + $TiO_2$ + $Al_2O_3$
$Al_2O_3$ flake + $SnO_2$
$Al_2O_3$ flake + $SnO_2$ + $TiO_2$
$Al_2O_3$ flake + $SnO_2$ + $Fe_2O_3$
$Al_2O_3$ flake + $SiO_2$
$Al_2O_3$ flake + $SiO_2$ + $TiO_2$
$Al_2O_3$ flake + $SiO_2$ + $TiO_2/Fe_2O_3$
$Al_2O_3$ flake + $SiO_2$ + $Fe_2O_3$
$Al_2O_3$ flake + $SiO_2$ + $TiO_2$ + $Fe_2O_3$
$Al_2O_3$ flake + $SiO_2$ + $TiO_2$ + $Fe_3O_4$
$Al_2O_3$ flake + $SiO_2$ + $TiO_2$ + $SiO_2$ + $TiO_2$
$Al_2O_3$ flake + $SiO_2$ + $Fe_2O_3$ + $SiO_2$ + $TiO_2$
$Al_2O_3$ flake + $SiO_2$ + $TiO_2/Fe_2O_3$ + $SiO_2$ + $TiO_2$

$Al_2O_3$ flake + $SiO_2$ + $TiO_2$ + $SiO_2$ + $TiO_2/Fe_2O_3$
$Al_2O_3$ flake + $SiO_2$ + $TiO_2$ + $SiO_2$
$Al_2O_3$ flake + $SiO_2$ + $TiO_2$ + $SiO_2/Al_2O_3$
$Al_2O_3$ flake + $SiO_2$ + $TiO_2$ + $Al_2O_3$
$Al_2O_3$ flake + $TiO_2$ + Prussian Blue
$Al_2O_3$ flake + $TiO_2$ + Carmine Red

**10.** $Al_2O_3$ flakes according to one or more of Claims 1 to 9 **characterised in that** the $Al_2O_3$ flakes are coated with $TiO_2$ in the rutile or anatase modification.

**11.** $Al_2O_3$ flakes according to one or more of Claims 1 to 10, **characterised in that** the $Al_2O_3$ flakes are coated with $TiO_2$ in the rutile modification.

**12.** $Al_2O_3$ flakes according to one or more of Claims 1 to 11, **characterized in that** the weight ratio of $Al_2O_3$ flakes and metal oxide coating is 40 : 60 to 90 : 10 based on the total pigment.

**13.** $Al_2O_3$ flakes according to one or more of Claims 6 to 12, **characterized in that** the coated $Al_2O_3$ flakes have a Flop index of $\geq 4$ (measured with spectrophotometer BYK-mac i).

**14.** Use of coated and uncoated $Al_2O_3$ flakes according to one or more of Claims 1 to 13 as base material for effect pigments, in formulations selected from paints, glaze compositions, coatings, automobile coatings, automotive finishing, industrial coatings, paints, powder coatings, printing inks, security printing inks, plastics, ceramic materials, cosmetics, glasses, paper, paper coating, toners for electrophotographic printing processes, seeds, greenhouse sheeting and tarpaulins, thermally conductive, self-supporting, electrically insulating, flexible sheets for the insulation of machines or devices, as absorber in the laser marking of paper and plastics, absorber in the laser welding of plastics, pigment pastes with water, organic and/or aqueous solvents, in pigment preparations and dry preparations, LIDAR and RADAR applications.

**15.** Formulations containing coated or uncoated $Al_2O_3$ flakes according to one or more of Claims 1 to 13 in amounts of 0.01-95 % by weight, based on the formulation as a whole.

**16.** Formulation containing coated or uncoated $Al_2O_3$ flakes according to one or more of Claims 1 to 13 **characterised in that** it contains at least one component selected from the group of water, polyols, polar and nonpolar oils, fats, waxes, film formers, polymers, copolymers, surfactants, free-radical scavengers, antioxidants, stabilisers, odour enhancers, silicone oils, emulsifiers, solvents, preservatives, thickeners, rheological additives, fragrances, colorants, effect pigments, UV absorbers, surface-active assistants and/or cosmetic active compounds, fillers, binders, pearlescent pigments, color pigments and organic dyes.

Fig. 1

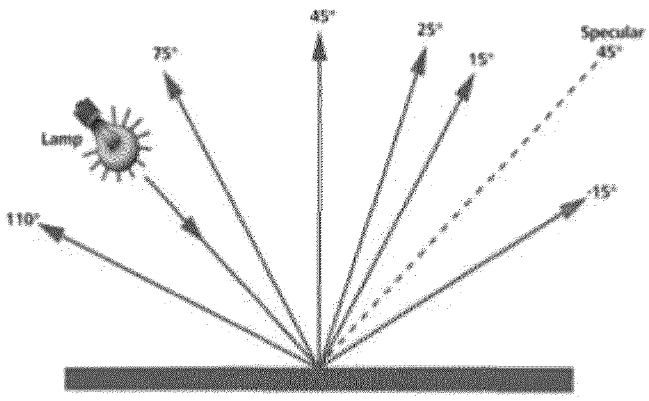

Europäisches Patentamt
European Patent Office
Office européen des brevets

# EUROPEAN SEARCH REPORT

Application Number
EP 24 21 9035

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/186225 A1 (LEE JUNG MIN [KR] ET AL) 23 July 2009 (2009-07-23) * paragraphs [0002], [0011] - [0014], [0025] - [0053] * * examples 1-5 * * table 1 * | 1-8, 13-16 | INV. C09C1/00 A61K8/19 C09D5/36 C09D11/02 |
| X | US 2019/338135 A1 (HAMM LUKAS [DE] ET AL) 7 November 2019 (2019-11-07) * paragraphs [0052] - [0169] * | 1-16 | |
| X | US 2019/169439 A1 (SCHOEN SABINE [DE] ET AL) 6 June 2019 (2019-06-06) * paragraphs [0008] - [0121] * | 1-16 | |
| X | US 2014/322536 A1 (SUZUKI RYUTA [JP] ET AL) 30 October 2014 (2014-10-30) * paragraphs [0010] - [0077] * | 1-16 | |

TECHNICAL FIELDS SEARCHED (IPC)

C09C
A61Q
C09G
C09D
A61K

The present search report has been drawn up for all claims

1

| Place of search | Date of completion of the search | Examiner |
|---|---|---|
| The Hague | 9 April 2025 | Marino, Emanuela |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........
& : member of the same patent family, corresponding document

EPO FORM 1503 03.82 (P04C01)

# ANNEX TO THE EUROPEAN SEARCH REPORT ON EUROPEAN PATENT APPLICATION NO. EP 24 21 9035

This annex lists the patent family members relating to the patent documents cited in the above-mentioned European search report. The members are as contained in the European Patent Office EDP file on
The European Patent Office is in no way liable for these particulars which are merely given for the purpose of information.

09-04-2025

| Patent document cited in search report | Publication date | Patent family member(s) | Publication date |
|---|---|---|---|
| US 2009186225 A1 | 23-07-2009 | CN 101146745 A | 19-03-2008 |
| | | EP 1871711 A1 | 02-01-2008 |
| | | JP 4825264 B2 | 30-11-2011 |
| | | JP 2008534417 A | 28-08-2008 |
| | | KR 100619248 B1 | 01-09-2006 |
| | | US 2009186225 A1 | 23-07-2009 |
| | | WO 2006101306 A1 | 28-09-2006 |
| US 2019338135 A1 | 07-11-2019 | CN 110436965 A | 12-11-2019 |
| | | EP 3564197 A1 | 06-11-2019 |
| | | US 2019338135 A1 | 07-11-2019 |
| US 2019169439 A1 | 06-06-2019 | EP 3211043 A1 | 30-08-2017 |
| | | GB 2549576 A | 25-10-2017 |
| | | JP 2017149946 A | 31-08-2017 |
| | | JP 2022082656 A | 02-06-2022 |
| | | JP 2024114764 A | 23-08-2024 |
| | | KR 20170099369 A | 31-08-2017 |
| | | US 2017240745 A1 | 24-08-2017 |
| | | US 2019169439 A1 | 06-06-2019 |
| | | US 2020123388 A1 | 23-04-2020 |
| US 2014322536 A1 | 30-10-2014 | CN 104130598 A | 05-11-2014 |
| | | CN 109825114 A | 31-05-2019 |
| | | EP 2799398 A2 | 05-11-2014 |
| | | EP 3366646 A1 | 29-08-2018 |
| | | ES 2684773 T3 | 04-10-2018 |
| | | JP 6412335 B2 | 24-10-2018 |
| | | JP 2014218425 A | 20-11-2014 |
| | | KR 20140130049 A | 07-11-2014 |
| | | KR 20210090127 A | 19-07-2021 |
| | | KR 20210090128 A | 19-07-2021 |
| | | TW 201505853 A | 16-02-2015 |
| | | US 2014322536 A1 | 30-10-2014 |
| | | US 2020231827 A1 | 23-07-2020 |
| | | US 2020231828 A1 | 23-07-2020 |
| | | US 2022243076 A1 | 04-08-2022 |
| | | US 2022251403 A1 | 11-08-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 57111239 A
- JP 3072572 A
- JP 4039362 A
- US 5702519 A
- EP 2799387 A
- EP 2799388 A
- WO 2006101306 A
- WO 2008026860 A1
- US 4038099 A
- US 5433779 A
- EP 0271767 A
- DE 3825702 A1
- DE 1467468
- DE 1959988
- DE 2009566
- DE 2214545
- DE 2215191
- DE 2244298
- DE 2313331
- DE 1522572
- DE 3137808
- DE 3137809
- DE 3151343
- DE 3151354
- DE 3151355
- DE 3211602
- DE 3235017
- EP 0045851 A
- EP 0106235 A
- DE 3151354 A
- DE 3235017 A
- DE 3334598 A
- EP 0090259 A
- EP 0634459 A
- WO 9957204 A
- WO 9632446 A
- US 5759255 A
- US 5571851 A
- WO 0192425 A
- WO 9813426 A


### Non-patent literature cited in the description


- **J.J. PONJEÉ**. *Philips Technical Review*, vol. 44 (3), 81
- **P.H. HARDING** ; **J.C. BERG**. *J. Adhesion Sci. Technol.*, vol. 11 (4), 471-493